# EUROPEAN PATENT SPECIFICATION

(11) **EP 1 463 455 B1**
(45) Date of publication and mention of the grant of the patent: **03.08.2005**
(21) Application number: 02785639.2
(22) Date of filing: 10.12.2002
(51) Int. Cl.: A61B 17/30, A61B 17/32

(54) **SURGICAL TOOL**
CHIRURGISCHES WERKZEUG
OUTIL CHIRURGICAL

(30) Priority: 09.01.2002 GB 0200393
(43) Date of publication of application: 06.10.2004
(73) Proprietor: Fulcrum (Medical Devices) Limited, London, Greater London W4 2QN (GB)
(72) Inventor: SINDING, Paul, London W4 2SA (GB)
(74) Representative: Grey, Ian Michael
(86) International application number: PCT/GB2002/005594
(87) International publication number: WO 2003/057053

(56) References cited:
- GB-A- 2 091 624
- US-A- 4 753 235
- US-A- 5 331 739

## Description

The present invention relates to a surgical tool and more specifically to a hand held surgical tool such as forceps or tweezers, scissors, pliers, scalpels and probes that are used, for example by ophthalmologists and plastic surgeons, during surgical or microsurgical procedures.

At present there exist many thousands of different hand-held surgical or microsurgical tools that are used for performing various different procedures on the human or animal body. Typically, each tool has a functional tip that is integrally formed with a handle from metal or other similar material. For example, forceps or tweezers are generally made from two pieces of metal that are welded together at one end to form an integral handle with opposable tips at the other end that are normally spaced from each other. When the arms are squeezed, they resiliently deform so that the tips are brought together to grip and/or manipulate tissue located between them.

A problem with tools of the type currently available is that the market is fragmented with a huge variety having a completely different handle design, weight and feel. The surgeon must therefore familiarise himself and be comfortable with many different instruments so that he/she can be fully competent in their particular area of expertise. Even if the surgeon only ever uses one sub-set of the many thousands of tools available, for example, an ophthalmologist will only use those tools possessing tips required for eye surgery such as cataract operations, they will still find that the handles of the majority of these tools are vastly different to each other. This situation is exacerbated when the surgeon uses a range of tools not all of which are made by the same company. The surgeon must then become familiar with the weight, feel and design of the tools manufactured by each company in addition to each individual tool produced by that company. This is obviously a disadvantage where surgery is concerned where there is little room for error and precise control and familiarity of all surgical equipment is vital.

Microsurgical tools are usually made from high-grade raw materials such as titanium metal to ensure that they have a reasonable lifespan despite repeated sterilization and handling. Although tools are often disposed of after a single use, many hospitals are reluctant to do this with conventional surgical tools because of their high cost. As the handle and tool tip are integrally formed, each tool is expensive as the handle is made from the same material as the tool tip. Although it is possible to make tools from other materials such as stainless steel, they degrade more quickly and must be disposed of sooner. This problem can be mitigated by manufacturing the handle and tool tip separately from different materials. However, the parts must then be welded or otherwise attached together with extreme accuracy and care which is difficult and time consuming. Therefore, no substantial reduction in cost is realised as a result of making the handle and tool tip separately from each other due to the problems of joining the components once they have been made.

US4 753 235 discloses a typical hand-held surgical tool comprising a tubular handle, and a planar tool element fixedly inserted in the distal end of said tubular handle.

It is an object of the present invention to overcome and/or alleviate the problems with conventional surgical and/or microsurgical tools discussed above.

According to the invention, there is provided a surgical tool comprising an elongate moulded handle having a body portion and a base portion and a substantially planar elongate tool member having a tip portion, the handle defining an elongate slot formed from corresponding channels in the body and base portions, each channel being configured to receive an edge of the tool member such that the tool member lies in a plane substantially at right angles to a plane along which the body portion and the base portion are joined.

The tool member must be adequately supported within the handle. By providing a slot formed by corresponding channels in both the body and base portions, the tool can be supported along at least part of its length disposed inside the handle thereby preventing any lateral movement of the tool.

In one embodiment, an actuator for controlling the tool member is mounted in an elongate aperture in the handle. The actuator enables control of tweezers, forceps and pliers. It is envisaged that other types of tool members not requiring an actuator can also be mounted in the handle, in which case the actuator will merely assist in retaining the tool member in the handle and will not perform another function.

Preferably, the actuator comprises a lever having a first end immovably mounted in the aperture in the body portion and a second end deflectable with respect to the first end and the body portion into the handle toward the base portion when pressure is applied to said second end.

Conveniently, a bridge divides the aperture in the body portion into two separate openings and a region of the lever between the first and second ends extends over the bridge. This enables the lever to deform in the region where it extends over the bridge when the second end of the lever is depressed, the first end being immovably mounted in the aperture in the body portion.

Preferably, the first end of the lever includes an elongate connecting member thereon dimensioned to be a snug fit in one opening between the body portion and the bridge to mount the lever to the body portion such that the region of the lever extending over the bridge deforms in response to the application of pressure to the second end of the lever.

The connecting member may be longer than the length of the opening and a cut-out may be advantageously formed in the underside of the body portion extending from the opening to receive the portion of the member that extends beyond the opening.

Advantageously, the upper surface of the lever is contoured to lie flush with the surface of the body portion.

The tool member preferably comprises forceps or tweezers having two opposable arms extending from an integral hub to the tip portion at the remote end of each arm, said arms being resiliently deformable to bring the tip portion of each arm into contact.

In a preferred embodiment, one arm is received and mounted in the channel in the base portion and the other arm is received and mounted in the channel in the body portion with the tip portion of said arms protruding from the handle through the aperture.

An extension of the channel formed in the body portion is conveniently formed in the lever such that the arm received in the channel in the body portion is also received in the channel in the lever such that when pressure is applied to the second end of the lever, the arm retained in the channel in the lever is urged towards the other arm to bring the end of the tip portions into contact, the resilience of the arm and of the lever urging it back to its original position when the pressure is released. It will be appreciated that only one arm is moved towards the other relative to the handle, rather than both arms as is the case with conventional forceps and tweezers. The fact that one arm remains stationary with respect to the handle provides the advantage that it is much easier to close the tool tip to grip very small items and with more accuracy as the stationary tool tip can be positioned and the other tool tip brought towards it by activation of the lever.

Preferably, the aperture through which the tip portion protrudes is formed in the base portion, said channel in the base portion terminating at the aperture.

In a preferred embodiment, the base portion includes an integrally formed upstanding nose section defining the end of the handle in which the aperture is formed.

Preferably, an end of the body portion includes a recess to receive the nose section of the base portion when the body and base portions are connected together.

The base portion and body portions include co-operating means to enable attachment of the body portion to the base portion.

Advantageously, the cooperating means comprises a pair of elongate rails depending from the length of the body portion for engagement with corresponding recesses in the base portion.

In a preferred embodiment, the tool member is unitary metal component formed from a flat sheet by, stamping, punching, laser cutting, water cutting, wire erosion or machining.

The handle is preferably contoured to enable it to be held in a pen-like grip between the thumb and forefinger and enable precise fingertip control. Most preferably, the body portion and the lever in the region of the second end of the lever to which pressure is applied is raised to form a smooth bump to enable the surgeon to clearly locate the part of the lever to which pressure must be applied to operate the tool member.

Preferably, the surgical tool of the present invention can be sterilised for re-use. However, because the tool member is now a much smaller component because it is mounted in a plastic handle rather than being integral with a handle formed from the same material, the tool of the present invention is much cheaper to manufacture. It is therefore envisaged that, because of the relatively low manufacturing cost, the tool of the present invention will be disposed of after a single use. This is a particular advantage as it avoids the need to sterilize the tool which may not be fully effective. It has also recently become apparent that vCJD (the human form of mad cow disease) is not completely destroyed as a result of carrying out standard sterilisation procedures. Not only does this mean that the same tool used to perform surgery on different people may infect all of them, but it also enables the possibility for cross-contamination of other tools being cleaned in the same cleaning and sterilisation process.

Embodiments of the present invention will now be described, by way of example only, with reference to the accompanying drawings, in which:

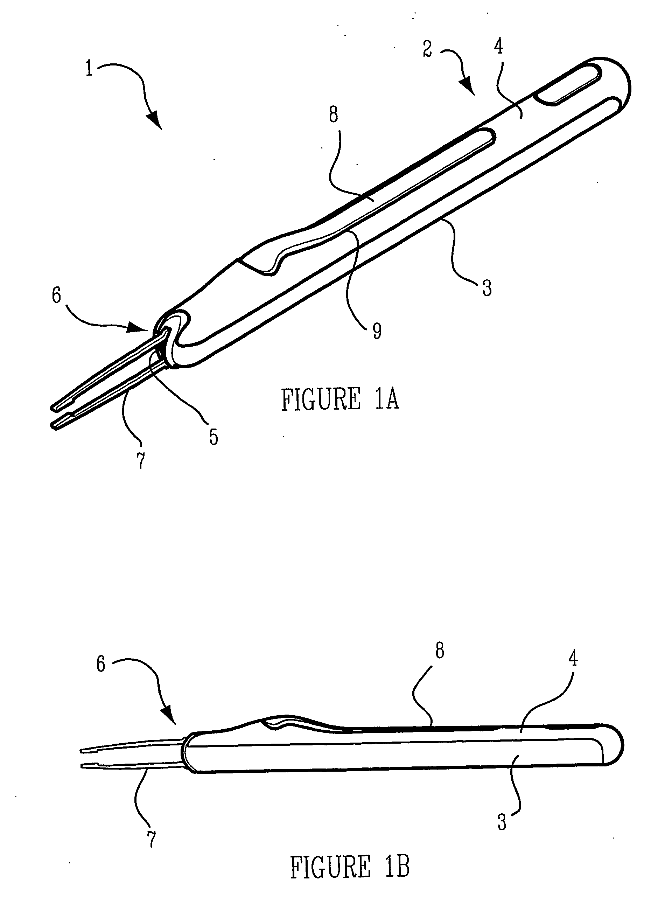

Figure 1A is a perspective view of a surgical tool according to the present invention;
Figure 1B is a side view of the surgical tool illustrated in Figure 1;
Figure 2A is a perspective view of the base portion of the handle of the surgical tool shown in Figures 1 and 2;
Figure 2B is a top plan view of the base portion shown in Figure 2A;
Figure 2C is a side view of the base portion shown in Figure 2A and 2B;
Figure 3A is a perspective view of the body portion of the handle of the surgical tool shown in Figures 1 and 2;
Figure 3B is a top plan view of the body portion shown in Figure 3A;
Figure 3C is a side view of the body portion shown in Figures 3A and 3B;
Figure 4A is a perspective view of the lever;
Figure 4B is a bottom plan view of the lever shown in Figure 1;
Figure 4C is a side view of the lever shown in Figure 4A and 4B;
Figure 5 is an exploded perspective view of the surgical tool illustrated in Figures 1 and 2;
Figure 6 is a perspective longitudinal cross-sectional view of the surgical tool illustrated in Figure 1;
Figure 7 is a perspective view of the tool member shown mounted within the handle of Figures 1A, 1B and Figure 6;
Figure 8A to 8C is a side view, a side sectional view along line A-A in a top plan view and the top plan view respectively, of a second embodiment of surgical tool according to the present invention;
Figure 9A to 9C is a side view, a side sectional view along line A-A in a top plan view and the top plan view respectively, of the surgical tool shown in Figures 8A to 8C but with a fixed rather than operable tool member mounted in the handle;
Figure 10A to 10C is a side view, a sectional side view along line A-A in a top plan view and the top plan view respectively, of a third embodiment of surgical tool according to the present invention; and
Figure 11A and 11B show examples of a number of different tool members that may be used with the surgical tools according to the embodiments of the present invention.

Referring now to the drawings, there is shown a surgical tool 1 according to an embodiment of the present invention comprising a handle 2 formed in two elongate halves from a first semi-cylindrical base portion 3 and a second semi-cylindrical body portion 4. An aperture 5 is provided in the end 6 of the handle 2 from which protrudes part of a surgical tool member 7 mounted within the handle 2. An actuator or lever 8 is mounted in an aperture 9 in the body portion 4 and lies flush with the contoured outer surface of the body portion 4.

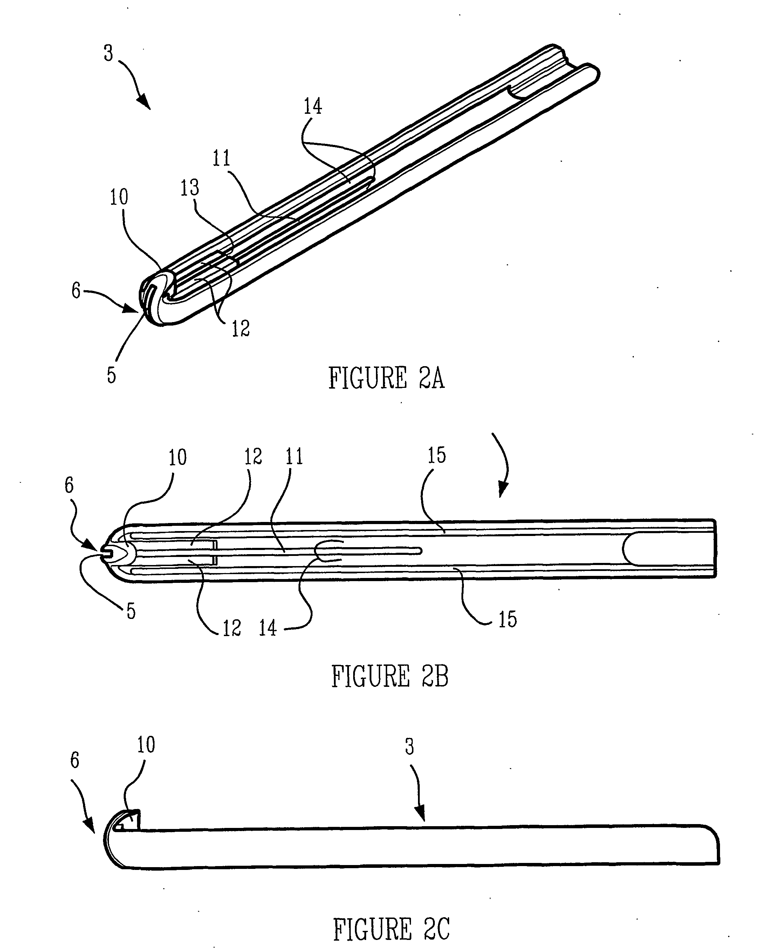

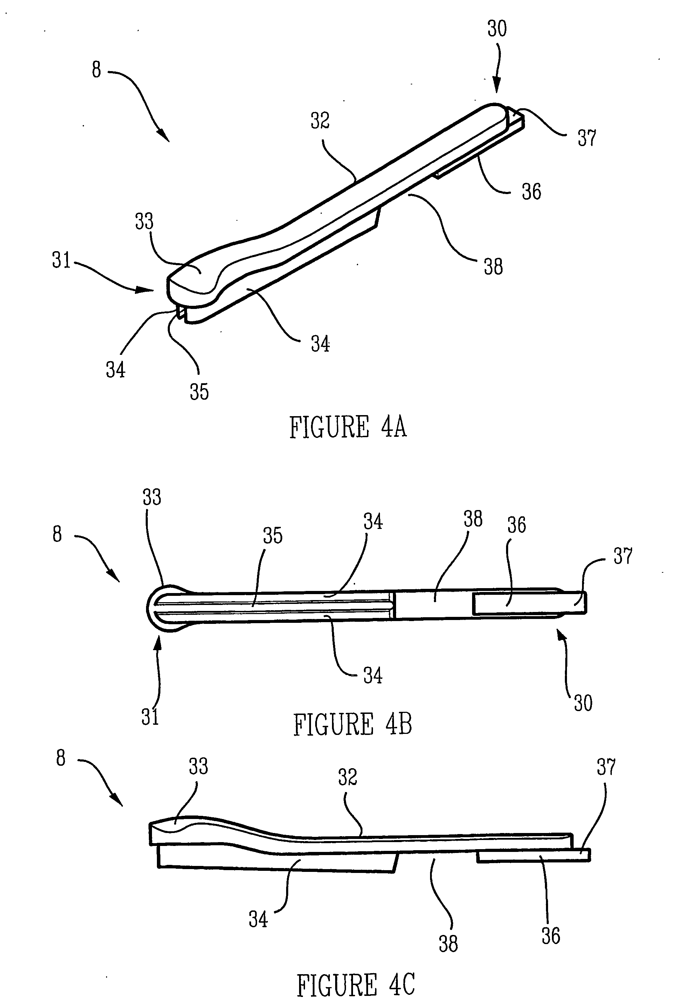

The base portion 3 will now be described in more detail with reference to Figures 2A to 2C from which it will be seen that the end 6 of the handle has a rounded nose portion 10 upstanding from the upper open part of the base portion 3. The aperture 5 takes the form of an arcuate slit in the nose portion 10. Extending rearwardly from and accessible via the aperture 5 is a longitudinal central channel 11 that extends for approximately half the length of the base portion 3. The channel 11 has raised side walls 12 for a short section immediately behind the aperture 9 which terminate in a shoulder 13 where the sides step down to lower side walls 14. The raised sidewalls 12 provide a deeper channel 11 than the channel 11 extending in the lower sidewalls 13, for reasons that will become apparent.

A pair of elongate recesses 15 are formed in the base portion 3 on either side and parallel to the central channel 11. These recesses 15 extend substantially the entire length of the base portion and enable connection of the base portion 3 to the body portion 4, as will be explained.

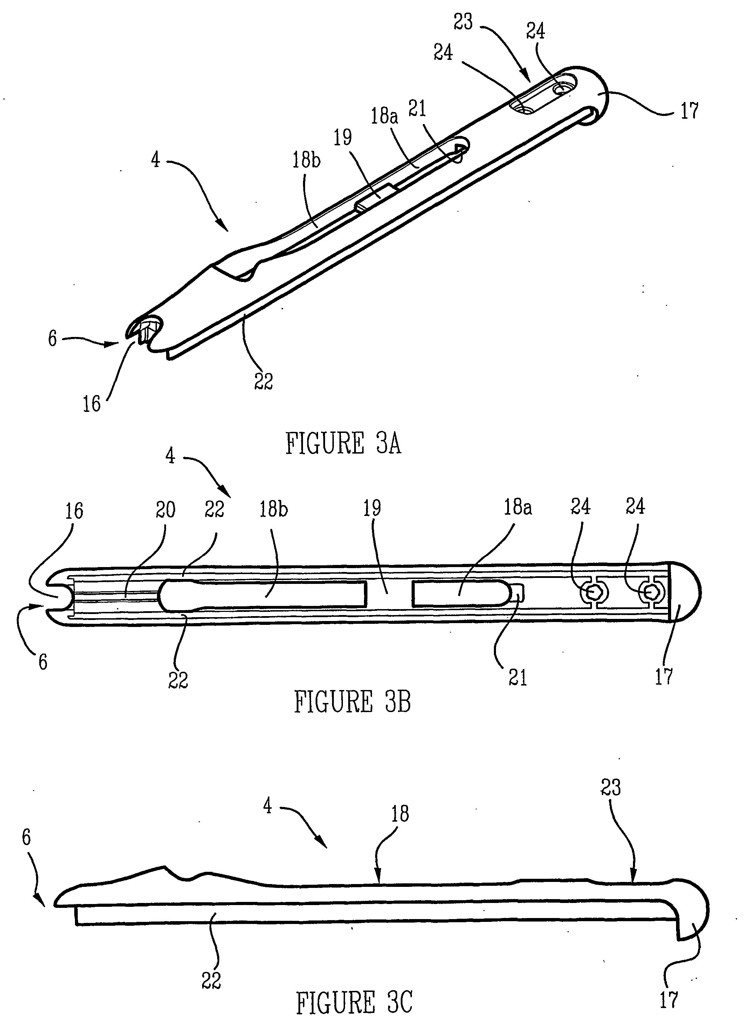

The body portion 4 will now be described with reference to Figures 3A to 3C from which it can be seen that it has a recess 16 to receive the nose portion 10 upstanding from the open surface of the base portion 3 when these components are assembled. The opposite end has a part spherical integrally formed cap 17 that closes the end of the handle 2 opposite to the end 6 in which the aperture 5 is formed. An elongate opening 18 divided into two parts 18a, 18b by a connecting member or bridge 19 is formed in the body portion 4 to receive and mount the lever 8 therein as will be explained. On the underside of the body portion 4, a longitudinal central channel 20 extends from the recess 16 to the elongate opening 18b. At the opposite end of the opening, a cut-out 21 or hollow is formed in the body portion 4.

A pair of rails 22 depend from the underside of the body portion 4 along its edges. These rails 22 co-operate and engage in the recesses 15 in the base portion to connect the base and body portions 3,4 together.

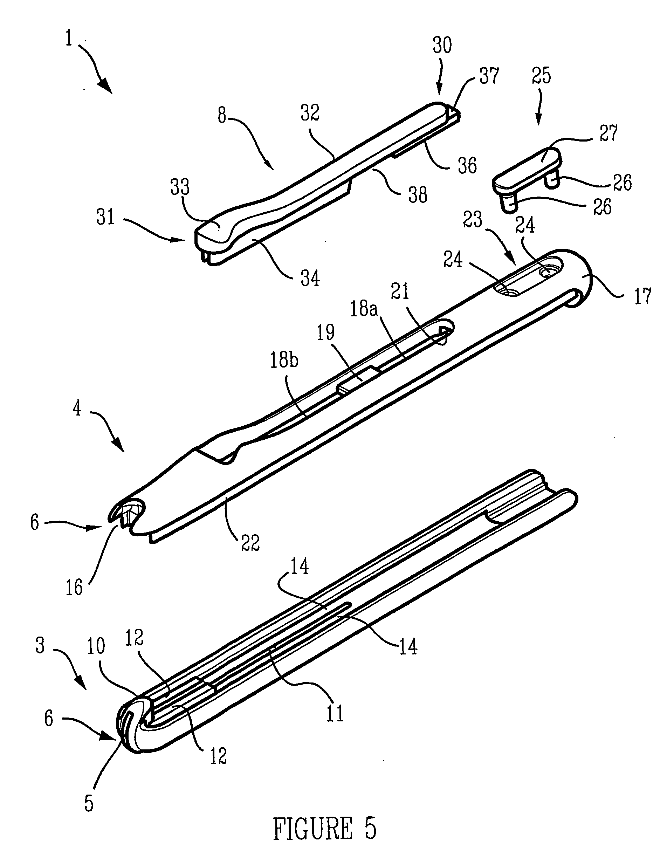

A groove 23 having a hole 24 at either end is also formed in the body portion 4 adjacent to the integrally formed cap 17. A member 25(see Figure 5) having two pins 26 connected by a head 27 fits in the groove 23 with each of the pins in one of the holes 24. The member 25 may be coloured differently to the rest of the handle to signify the type of tool member mounted therein. Alternatively, or in addition, a logo or other information may be presented on the head 27. The member 25 may be easily removed and/or replaced as required.

The lever 8 will now be described with reference to Figures 4A to 4C. The lever 8 is a unitary plastics component having a first end 30 for attaching the lever 8 to the body portion 4 and a second end 31 to which pressure may be applied to actuate a tool member 7 located in the handle 2. The lever 8 has an upper body part 32 extending between the first and second ends 30,31, and having an enlarged raised portion to form an operating button 33 at the second end 31. Extending downwardly from the upper body part 32 in the region of the second end 31 is a pair of parallel elongate wall portions 34 defining a channel 35 therebetween. The wall portions 34 extend rearwardly towards the first end 30 but terminate approximately two-thirds along the length of the upper body part 32. A locating member 36 for attaching the lever 8 to the body portion 4 is disposed on the underside of the first end 30 and protrudes slightly beyond the end of the upper body part 32 to form a tab 37. A gap 38 is formed between the locating member 36 and the parallel elongate walls 34 which is of a similar length to the length of the connecting member or bridge 19 formed in the body portion 4.

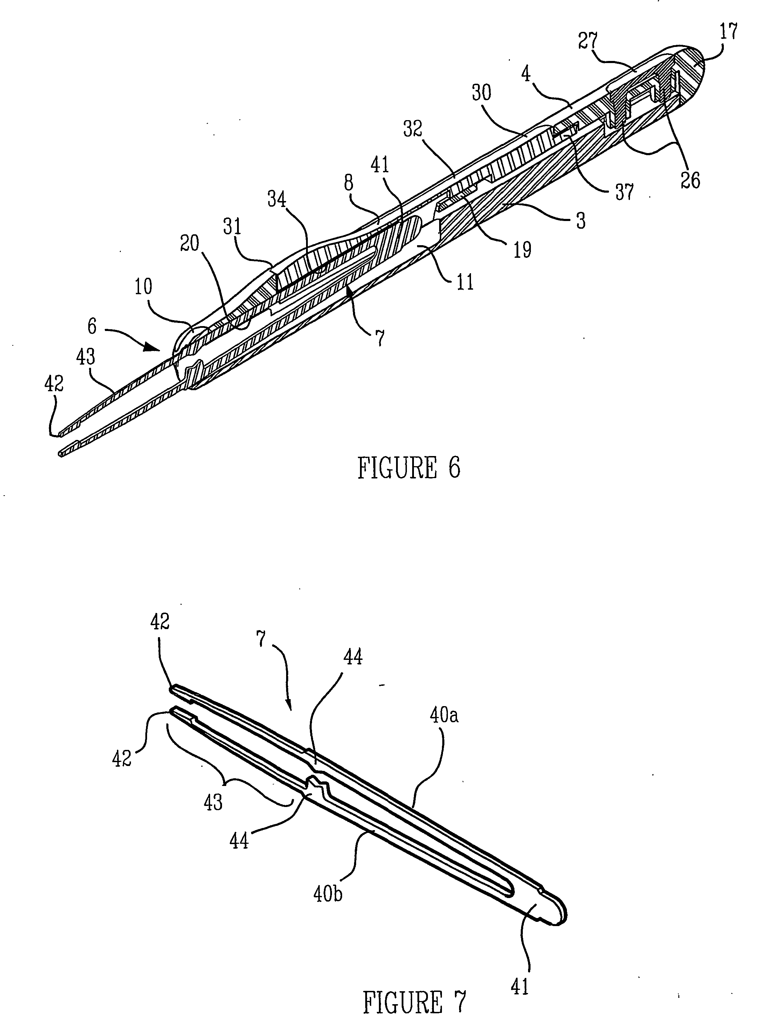

A number of different surgical tool members may be mounted within the handle 2. An example of one tool member 7 is illustrated in Figure 7. This tool member 7 replaces a conventional pair of forceps or tweezers and has a pair of opposed elongate arms 40a, 40b extending from a hub 41. The tips 42 of the arms 40a,40b at the end of the tip portions 43 may be closed together by squeezing them in the direction indicated by X. Facing co-operating protrusions 44 are formed on each arm 40a, 40b which engage to prevent excess pressure from being applied to the arms 40a,40b when the tips 42 are closed. The tool member 7 is formed in one unitary piece from a flat sheet of metal such as titanium and can be manufactured using a relatively low cost process relative to the metal turning processes currently employed. Such processed may include punching, stamping, laser cutting, water cutting, wire erosion or a machining process. In addition, as the tool member 7 and the handle 2 are formed as separate components from different materials, the use of high-grade expensive materials can be kept to a minimum, the tool member 7 being much smaller than a conventional tool having an integrally formed handle.

Assembly of the tool will now be described with reference to the exploded perspective view of Figure 5. A selected tool member such as the one illustrated in Figure 7 (but not shown in Figure 5) is first attached to the base portion 3 by passing the tool tip through the aperture 5 and by pressing the tool member into the channel 11 so that it is held thereby substantially all along its length within the base portion 3. Next, the body portion 4 is lowered onto the base portion 3 in the direction indicated by arrow A so that each rail 22 locates in the recess 15 in the base portion 3. The recess 16 in the body portion 4 surrounds the nose portion 10 and the cap 17 closes the end of the handle 2 remote from the aperture 5. It will be appreciated that part of the tool member 7 not retained in the channel 11 in the base portion locates in the channel 20 in the body portion 4 during this step. The body portion 4 and base portion 3 may be sealed and joined together using resin or adhesive material.

The lever 8 is now attached to the body portion 4 by lowering it into the aperture 9 in the direction of arrow B. The tab 37 on the locating member 36 locates within the cut-out 21 in the body portion 4 and the locating member 36 fits snugly in the aperture 19a. The connecting member or bridge 19 locates in the gap 38 between the wall portions 34 and the locating member 36. The channel 34 formed by the wall portions 34 forms an extension of the channel 20 in the body portion 4 and also receives part of the tool member 7 and the corresponding channels 11,20,34 in the base portion, the body portion and in the lever 8 together form a slot in the handle to receive a tool member.

The side sectional perspective view of Figure 6 shows how the tool member 7 of Figure 7 is retained in the channels 11, 20, 34 formed by the base portion 3, the body portion 4 and the lever 8, respectively. It will be appreciated that the tool is a tight sliding fit in the channel 11 in the base portion 3 and the channel 34 in the lever 8 and is firmly held thereby. The channel 20 is slightly wider than channels 11, 34 for reasons which will become apparent. As the tool 7 is supported within the channels 11,20,34 along substantially its entire length within the handle 2, lateral movement of the tool 7 is prevented. This is particularly important with the forcep or tweezer tool illustrated in Figure 7 as any lateral movement of one arm 40a with respect to the other arm 40b would result in the tips 42 not being in alignment. It will be appreciated that the raised sidewalls 12 of the channel 11 provide additional lateral support for the tool member in the region immediately behind the aperture 5.

The operation of the surgical tool 1 having the tool member 7 mounted therein will now be explained with reference to Figure 6. When pressure is applied to the operating button 33 in the direction indicated by arrow Y, the upper portion 32 of the lever 8 deforms in the region extending over the connecting portion 19 and deflects inwardly toward the base portion 3, the first end 30 of the lever 8 being firmly held as a result of engagement of the locating member 36 in the aperture 19a and the tab 37 in the cut-out 21. As the arm 40a of the tool member 7 is received in the channel 34, the arm 40a is urged towards the other arm 40b thereby closing the tool tips 42. When pressure on the operating button 33 is released, the resilience of the arm 40a and the resilience of the lever 8 causes it to return to its original position thereby separating the tool tips 42. It will be appreciated that the arm 40a moves out of the channel 20 in the body portion 4 when it moves toward the other arm 40b. Therefore, the arm 40a is a slightly looser fit in the channel 20, achieved by making it wider than channel 11, 34.

It will be appreciated that the outer surface of the base and body portions 3,4 are rounded to provide a comfortable gripping surface. The outer surface of the body part 32 of the lever 8 is also rounded and fits flush with the outer surface of the body portion 4. The handle 2 is ergonomically designed to suit being held in a pen-like grip between the thumb and forefinger for precision control of the tool 1.

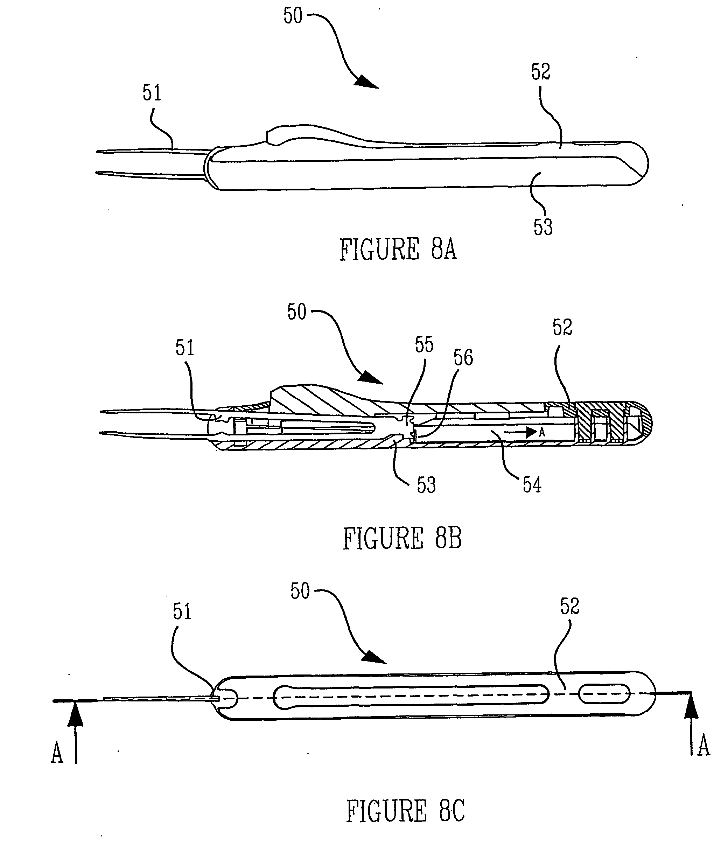

A second embodiment of the surgical tool according to the present invention will now be described with reference to Figures 8A to 8C. This embodiment is similar to the first embodiment and so those features which are identical will not be described again. In this embodiment, the surgical tool comprises a handle 50 which has a similar construction to the handle 2 of the first embodiment and supports a tool member 51 in the same way. However, the two elongate halved 52,53 of the handle 50 are formed so as to create a cavity 54 in a region behind the tool member 51. The end 55 of the tool member 51 is held in a channel similar to channel 11 of the first embodiment. However, the tool member is prevented from sliding into the cavity 54 by a frangible wall section or stop 56 upstanding from the elongate half 53 of the handle 50. The end of the tool member lies 51 lies in contact with the frangible wall section 56, as most clearly shown in the side sectional view of Figure 8B.

When the surgeon has finished using the tool, the tool member 51 can be retracted inside the handle 50 for secure disposal of the entire tool. The retraction of the tool member 51 can be achieved by striking the tool member 51 firmly against a surface with a force sufficient to cause the stop 56 to break. The tool member 51 is then free to slide, in the direction of arrow A in Figure 8B into the cavity 54.

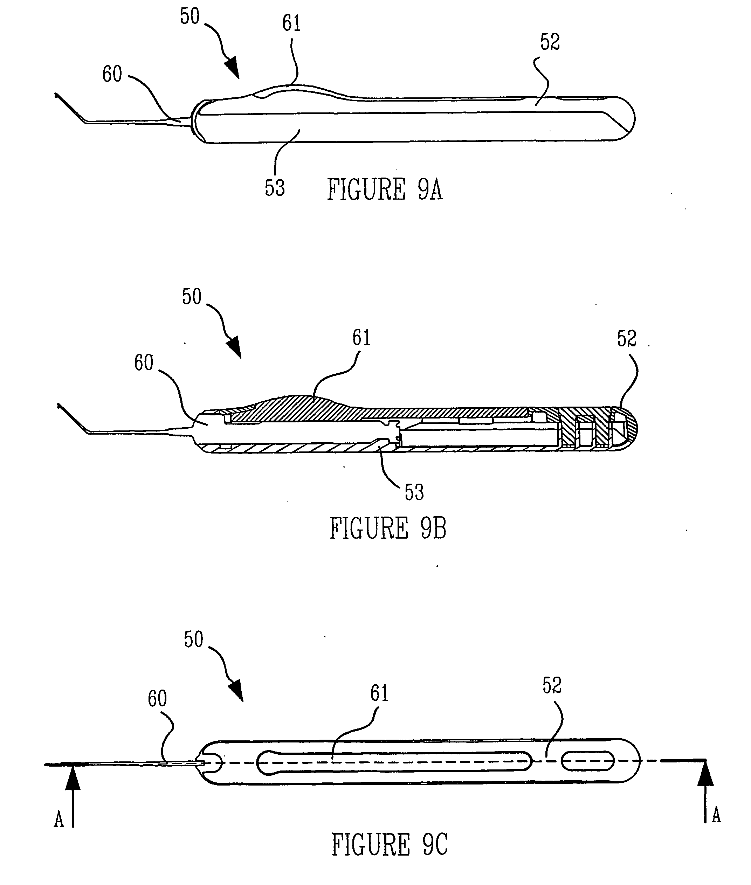

Figures 9A to 9C show the same surgical tool and handle 50 as the second embodiment described with reference to Figures 8A to 8C. However, in this embodiment, the handle 50 is shown with another type of tool member 60 mounted therein. This tool member 60 is of the static type, by which is meant that it is not operated by activating a lever as is required with tweezers or forceps such as those described with reference to Figure 7. An example of such a tool member is a probe or scalpel. The lever 61 shown in this embodiment is therefore redundant and merely serves to retain the tool member 60 firmly within the handle 50.

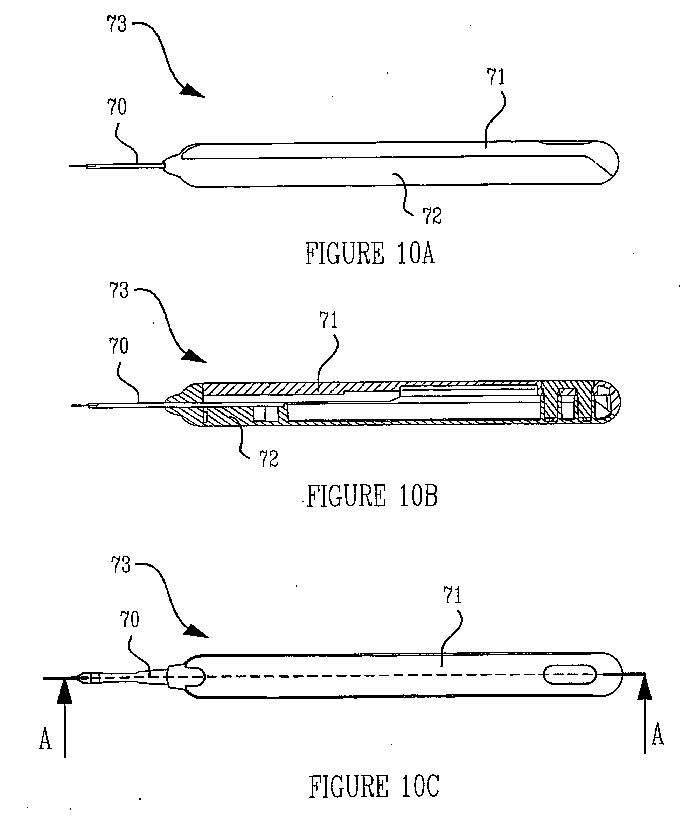

Figures 10A to 10C show another embodiment in which the actuator or lever 8 described with reference to the first embodiment is omitted, the tool member 70 being retained between the two elongate halves 71,72 of the handle 73. It will be appreciated that the handle of this embodiment can only be used with tools of the static type.

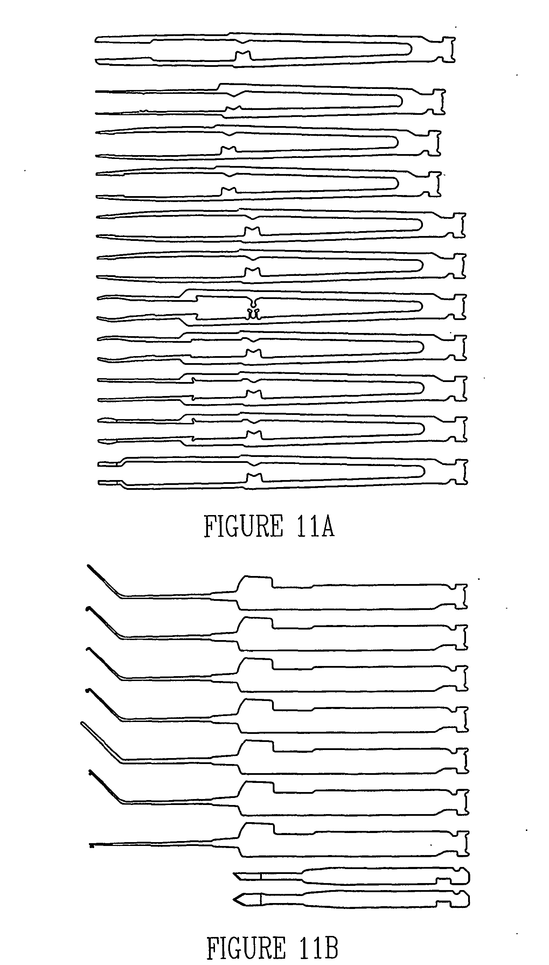

Figure 11A illustrates examples of eleven different tool members that may be mounted within the handle of any of the embodiments of the invention. Figure 11B illustrates examples of nine different static type tool members that may alternatively be mounted within the handle of any of the embodiments of the invention.

It will be apparent that the present invention provides the surgeon with one familiar handle shape that allows all types of instrument tool tips to be held. Some embodiments of the invention also provide means for retracting the tool member into the handle after use, thereby reducing the threat of injury or contamination.

Many modifications and variations to the invention falling within the terms of the following claims will be apparent to those skilled in the art.

## Claims

1. A surgical tool (1) comprising an elongate moulded handle (2) having a body portion (4) and a base portion (3) and a substantially planar elongate tool member (7) having a tip portion (43), the body (4) and base portions (3) being fixed relative to each other to form the handle (2) when the tool (1) is assembled, the handle (2) defining an elongate slot (5) formed from corresponding channels (20,11) in the body and base portions (4,3), each channel (20,11) being configured to receive an edge of the tool member (7) such that the tool member (7) it lies in a plane substantially at right angles to a plane along which the body portion (4) and the base portion (3) are fixed relative to each other.

2. A surgical tool (1) according to claim 1 wherein an actuator (8) for controlling the tool member (7) is mounted in an elongate aperture (5) in the handle (2).

3. A surgical tool (1) according to claim 2 wherein the actuator comprises a lever (8) having a first end (30) immovably mounted in the aperture (9) in the body portion (4) and a second end (31) deflectable with respect to the first end (30) and the body portion (4) into the handle (2) toward the base portion (3) when pressure is applied to said second end (31).

4. A surgical tool (1) according to claim 3 wherein a bridge (19) divides the aperture (9) in the body portion (4) into two separate openings (18a,18b) and a region of the lever (8) between the first and second ends (30,31) extends over the bridge (19).

5. A surgical tool (1) according to claim 4 wherein said first end (30) of the lever (8) includes an elongate connecting member (36) thereon dimensioned to be a snug fit in one opening (18a) between the body portion (4) and the bridge (19) to mount the lever (8) to the body portion (4) such that the region of the lever (8) extending over the bridge (19) deforms in response to the application of pressure to the second end (31) of the lever (8).

6. A surgical tool (1) according to claim 5 wherein the connecting member (36) is longer than the length of the opening (18a) and a cut-out (21) is formed in the underside of the body portion (4) extending from the opening (18a) to receive the portion of the member (36) that extends beyond the opening (18a).

7. A surgical tool (1) according to any of claims 3 to 6 wherein the upper surface of the lever (8) is contoured to lie flush with the surface of the body portion (4).

8. A surgical tool (1) according to any preceding claim, wherein the tool member (7) comprises forceps or tweezers having two opposable arms (40a,40b) extending from an integral hub (41) to the tip portion (43) at the remote end of each arm (40a,40b), said arms (40a,40b) being resiliently deformable to bring the tip portion (43) on each arm (40a,40b) into contact.

9. A surgical tool (1) according to claim 8 wherein one arm (40a) is received and mounted in the channel (11) in the base portion (3) and the other arm (40b) is received and mounted in the channel (20) in the body portion (4) with the tip portion (43) of said arms (40a,40b) protruding from the handle (2) through the aperture (5).

10. A surgical tool (1) according to claim 9 wherein an extension of the channel (20) formed in the body portion (4) is formed in the lever (8) such that the arm (40b) received in the channel (20) in the body portion (4) is also received in the channel (35) in the lever (8) such that when pressure is applied to the second end (31) of the lever (8), the arm (40b) retained in the channel (35) in the lever (8) is urged towards the other arm (40a) to bring the tip portions (43) into contact, the resilience of the arm (40b) and of the lever (8) urging it back to its original position when the pressure is released.

11. A surgical tool (1) according to any preceding claim, wherein the aperture (5) through which the tool tip (43) protrudes is formed in the base portion (3), said channel (11) in the slot in the base portion (3) terminating at the aperture (5).

12. A surgical tool (1) according to claim 11, wherein the base portion (3) includes an integrally formed upstanding nose section (10) defining the end of the handle (6) in which the aperture (5) is formed.

13. A surgical tool (1) according to claim 12 wherein an end of the body portion (4) includes a recess (16) to receive the nose section (10) of the base portion (3).

14. A surgical tool (1) according to any preceding claim, including co-operating means (15,22) to attach the body portion (4) to the base portion (3).

15. A surgical tool (1) according to claim 14 wherein the cooperating means (15,22) comprises a pair of elongate rails (22) depending from the length of the body portion (15) for engagement with corresponding recesses (15) in the base portion (3).

16. A surgical tool (1) according to any preceding claim, wherein the tool member (7) is unitary metal component formed from a flat sheet by, stamping, punching, laser cutting, water cutting, wire erosion or machining.

17. A surgical tool (1) according to any preceding claim, wherein the handle (2) is contoured to enable it to be held in a pen-like grip between the thumb and forefinger and enable precise fingertip control.

18. A surgical tool (1) according to any preceding claim, wherein the handle (2) defines a cavity (54) therein and a frangible wall section (56) to prevent the tool member (7) from sliding into the cavity (54).

19. A surgical tool (1) according to claim 18 wherein the frangible wall (56) is configured such that when force is applied to the tool member (7) in a longitudinal direction toward the handle (2), the frangible wall (56) breaks to allow the tool member (7) to retract into the cavity (54).

20. A surgical tool (1) according to claim 17 wherein the base portion (3) or the body portion (4) is coloured to signify the type of tool member (7) contained in the handle (2).

21. A surgical tool (1) according to any preceding claim, wherein the tool (1) can be sterilised for re-use.

22. A surgical tool (1) according to any preceding claim, wherein the moulded handle (2) is formed from a plastics material.

## Patentansprüche

1. Chirurgisches Instrument (1), das einen länglichen geformten Griff (2) mit einem Körperabschnitt (4) und einem Basisabschnitt (3) und ein im Wesentlichen planares längliches Instrumentenelement (7) mit einem Spitzenabschnitt (43) umfasst, wobei der Körper- (4) und der Basisabschnitt (3) relativ zueinander befestigt sind, um den Griff (2) zu bilden, wenn das Instrument (1) zusammengebaut ist, wobei der Griff (2) einen länglichen Schlitz (5) definiert, der von entsprechenden Rinnen (20, 11) in dem Körper- und dem Basisabschnitt (4, 3) gebildet wird, wobei jede Rinne (20, 11) zum Aufnehmen eines Rands des Instrumentenelements (7) konfiguriert ist, so dass das Instrumentenelement (7) in einer Ebene liegt, die im Wesentlichen im rechten Winkel zu einer Ebene ist, längs derer der Körperabschnitt (4) und der Basisabschnitt (3) relativ zueinander befestigt sind.

2. Chirurgisches Instrument (1) nach Anspruch 1, bei dem ein Steller (B) zum Steuern des Instrumentenelements (7) in einer länglichen Ausnehmung (5) in dem Griff (2) montiert ist.

3. Chirurgisches Instrument (1) nach Anspruch 2, bei dem der Steller einen Hebel (8) mit einem ersten Ende (30), das unbeweglich in der Ausnehmung (9) in dem Körperabschnitt (4) montiert ist, und einem zweiten Ende (31) umfasst, das mit Bezug auf das erste Ende (30) und den Körperabschnitt (4) in Richtung auf den Basisabschnitt (3) in den Griff (2) abgelenkt werden kann, wenn Druck auf das genannte zweite Ende (31) ausgeübt wird.

4. Chirurgisches Instrument (1) nach Anspruch 3, bei dem eine Brücke (19) die Ausnehmung (9) in dem Körperabschnitt (4) in zwei separate Öffnungen (18a, 18b) unterteilt und eine Region des Hebels (8) zwischen dem ersten und dem zweiten Ende (30, 31) über die Brücke (19) verläuft.

5. Chirurgisches Instrument (1) nach Anspruch 4, bei dem das genannte erste Ende (30) des Hebels (8) ein längliches verbindungselement (36) daran aufweist, das dimensioniert ist, um satt anliegend in einer Öffnung (18) zwischen dem Körperabschnitt (4) und der Brücke (19) zu sitzen, um den Hebel (8) an dem Körperabschnitt (4) zu montieren, so dass die über die Brücke (19) verlaufende Region des Hebels (8) sich in Reaktion auf das Ausüben von Druck auf das zweite Ende (31) des Hebels (8) verformt.

6. Chirurgisches Instrument (1) nach Anspruch 5, bei dem das Verbindungselement (36) länger als die Länge der Öffnung (18a) ist und in der Unterseite des Körperabschnitts (4) ein sich ab der Öffnung (18a) erstreckender Ausschnitt (21) gebildet ist zum Aufnehmen des Abschnitts des Elements (36), der über die Öffnung (18a) hinaus verläuft.

7. Chirurgisches Instrument (1) nach einem der Ansprüche 3 bis 6, bei dem die Oberseite des Hebels (8) konturiert ist, um mit der Oberfläche des Körperabschnitts (4) bündig zu sein.

8. Chirurgisches Instrument (1) nach einem der vorhergehenden Ansprüche, bei dem das Instrumentenelement (7) eine Zange oder Pinzette mit zwei einander entgegensetzbaren Armen (40a, 40b) umfasst, die von einer einstückigen Nabe (41) zu dem Spitzenabschnitt (43) am fernen Ende jedes Arms (40a, 40b) verlaufen, wobei die genannten Arme (40a, 40b) elastisch verformbar sind, um die Spitzenabschnitte (43) an den Armen (40a, 40b) jeweils miteinander in Kontakt zu bringen.

9. Chirurgisches Instrument (1) nach Anspruch 8, bei dem ein Arm (40a) in der Rinne (11) in dem Basisabschnitt (3) aufgenommen und montiert ist und der andere Arm (40b) in der Rinne (20) in dem Körperabschnitt (4) aufgenommen und montiert ist, wobei der Spitzenabschnitt (43) der genannten Arme (40a, 40b) durch die Ausnehmung (5) aus dem Griff (2) herausragt.

10. Chirurgisches Instrument (1) nach Anspruch 9, bei dem eine in dem Körperabschnitt (4) gebildete Verlängerung der Rinne (20) so in dem Hebel (8) ausgebildet ist, dass der in der Rinne (20) in dem Körperabschnitt (4) aufgenommene Arm (40b) auch in der Rinne (35) in dem Hebel (8) aufgenommen ist, so dass der in der Rinne (35) in dem Hebel (8) gehaltene Arm (40b), wenn Druck auf das zweite Ende (31) des Hebels (8) ausgeübt wird, in Richtung auf den anderen Arm (40a) gedrängt wird, um die Spitzenabschnitte (43) in Kontakt zu bringen, wobei die Elastizität des Arms (40b) und des Hebels (8) ihn wieder in seine Ausgangsposition zurüclzdrängt, wenn der Druck weggenommen wird.

11. Chirurgisches Instrument (1) nach einem der vorhergehenden Ansprüche, bei dem die Ausnehmung (5), durch welche die Instrumentenspitze (43) herausragt, in dem Basisabschnitt (3) gebildet ist, wobei die genannte Rinne (11) in dem Schlitz in dem Basisabschnitt (3) an der Ausnehmung (5) endet.

12. Chirurgisches Instrument (1) nach Anspruch 11, bei dem der Basisabschnitt (3) einen einstückig ausgebildeten hochstehenden Nasenabachnitt (10) hat, der das Ende des Griffes (6) definiert, in dem die Ausnehmung (5) ausgebildet ist.

13. Chirurgisches Instrument (1) nach Anspruch 12, bei dem ein Ende des Körperabschnitts (4) eine Aussparung (16) zum Aufnehmen des Nasenabschnitts (10) des Basisabschnitts (3) hat.

14. Chirurgisches Instrument (1) nach einem der vorhergehenden Ansprüche, das zusammenwirkende Mittel (15, 22) zum Anbringen des Körperabschnitts (4) an dem Basisabschnitt (3) hat.

15. Chirurgisches Instrument (1) nach Anspruch 14, bei dem die zusammenwirkenden Mittel (15, 22) ein Paar länglicher Schienen (22), die von der Länge des Körperabschnitts (15) abhängen, zum Eingriff in entsprechende Aussparungen (15) in dem Basisabschnitt (3) umfassen.

16. Chirurgisches Instrument (1) nach einem der vorhergehenden Ansprüche, bei dem das Instrumentenelement (7) ein einstückiges Metallteil ist, das durch Stanzen, Lochen, Laserschneiden, Wasserstrahlschneiden, Drahterodieren oder spanhebendes Bearbeiten aus einer flächigen Platte hergestellt ist.

17. Chirurgisches Instrument (1) nach einem der vorhergehenden Ansprüche, bei dem der Griff (2) so konturiert ist, dass er wie ein Schreibstift zwischen Daumen und Zeigefinger gehalten werden kann und präzise Fingerspitzenkontrolle ermöglicht.

18. Chirurgisches Instrument (1) nach einem der vorhergehenden Ansprüche, bei dem der Griff (2) in sich eine Höhlung (54) und einen bruchfähigen Wandabschnitt (56) zum Verhindern, dass das Werkzeugelement (7) in die Höhlung (54) hineinrutscht, definiert.

19. Chirurgisches Instrument (1) nach Anspruch 18, bei dem der bruchfähige Wandabschnitt (56) so konfiguriert ist, dass, wenn Kraft in einer Längsrichtung auf den Griff (2) zu auf das Instrumentenelement (7) ausgeübt wird, der bruchfähige Wandabschnitt (56) bricht, damit das Instrumentenelement (7) in die Höhlung (54) zurückgeschoben werden kann.

20. Chirurgisches Instrument (1) nach Anspruch 17, bei dem der Basisabschnitt (3) oder der Körperabschnitt (4) farbig ist, um den Typ des in dem Griff (2) enthaltenen Instrumentenelements (7) zu bezeichnen.

21. Chirurgisches Instrument (1) nach einem der vorhergehenden Ansprüche, bei dem das Instrument (1) zur Wiederverwendung sterilisiert werden kann.

22. Chirurgisches Instrument (1) nach einem der vorhergehenden Ansprüche, bei dem der geformte Griff (2) aus einem Kunststoffmaterial hergestellt ist.

## Revendications

1. Outil chirurgical (1) comprenant une poignée moulée allongée (2) possédant une portion de corps (4) et une portion de base (3) et un élément d'outil allongé essentiellement plan (7) possédant une portion de pointe (43), les portions de corps (4) et de base (3) étant fixées l'une par rapport à l'autre pour former la poignée (2) lorsque l'outil (1) est assemblé, la poignée (2) définissant une fente allongée (5) formée à partir de canaux correspondants (20, 11) dans les portions de corps et de base (4, 3), chaque canal (20, 11) étant configuré pour recevoir un bord de l'élément d'outil (7) de sorte que l'élément d'outil (7) se trouve dans un plan essentiellement à angle droit à un plan le long duquel la portion de corps (4) et la portion de base (3) sont fixées l'une par rapport à l'autre.

2. Outil chirurgical (1) selon la revendication 1, dans lequel un actionneur (8) pour le contrôle de l'élément d'outil (7) est monté dans une ouverture allongée (5) dans la poignée (2).

3. Outil chirurgical (1) selon la revendication 2, dans lequel l'actionneur comprend un levier (8) possédant une première extrémité (30) montée à demeure dans l'ouverture (9) dans la portion de corps (4) et une deuxième extrémité (31) pouvant être déviée par rapport à la première extrémité (30) et la portion de corps (4) dans la poignée (2) vers la portion de base (3) lorsqu'une pression est appliquée sur ladite deuxième extrémité (31).

4. Outil chirurgical (1) selon la revendication 3, dans lequel un pont (19) divise l'ouverture (9) dans la portion de corps (4) en deux ouvertures séparées (18a, 18b) et une région du levier (8) entre les première et deuxième extrémités (30, 31) s'étend au-dessus du pont (19).

5. Outil chirurgical (1) selon la revendication 4, dans lequel ladite première extrémité (30) du levier (8) comprend un élément de connexion allongé (36) sur celui-ci dimensionné pour s'ajuster étroitement dans une ouverture (18a) entre la portion de corps (4) et le pont (19) pour monter le levier (8) sur la portion de corps (4) de sorte que la région du levier (8) s'étendant au-dessus du pont (19) se déforme en réponse à l'application d'une pression sur la deuxième extrémité (31) du levier (8).

6. Outil chirurgical (1) selon la revendication 5, dans lequel l'élément de connexion (36) est plus long que la longueur de l'ouverture (18a) et une découpe (21) est formée dans la face inférieure de la portion de corps (4) s'étendant depuis l'ouverture (18a) pour recevoir la portion de l'élément (36) qui s'étend au-delà de l'ouverture (18a).

7. Outil chirurgical (1) selon l'une quelconque des revendications 3 à 6, dans lequel la surface supérieure du levier (8) est pourvue de contours pour se trouver à fleur de la surface de la portion de corps (4).

8. Outil chirurgical (1) selon l'une quelconque des revendications précédentes, dans lequel l'élément d'outil (7) comprend des forceps ou des précelles possédant deux bras opposables (40a, 40b) s'étendant depuis un moyeu intégral (41) vers la portion de pointe (43) à l'extrémité distante de chaque bras (40a, 40b), lesdits bras (40a, 40b) pouvant être déformés de manière élastique pour amener la portion de pointe (43) sur chaque bras (40a, 40b) en contact.

9. Outil chirurgical (1) selon la revendication 8, dans lequel un bras (40a) est reçu et monté dans le canal (11) dans la portion de base (3) et l'autre bras (40b) est reçu et monté dans le canal (20) dans la portion de corps (4) avec la portion de pointe (43) desdits bras (40a, 40b) faisant saillie depuis la poignée (2) à travers l'ouverture (5).

10. Outil chirurgical (1) selon la revendication 9, dans lequel un prolongement du canal (20) formé dans la portion de corps (4) est formé dans le levier (8) de sorte que le bras (40b) reçu dans le canal (20) dans la portion de corps (4) est également reçu dans le canal (35) dans le levier (8) de sorte que lorsqu'une pression est appliquée sur la deuxième extrémité (31) du levier (8), le bras (40b) retenu dans le canal (35) dans le levier (8) est poussé vers l'autre bras (40a) pour amener les portions de pointe (43) en contact, la résilience du bras (40b) et du levier (8) le repoussant dans sa position d'origine lorsque la pression est relâchée.

11. Outil chirurgical (1) selon l'une quelconque des revendications précédentes, dans lequel l'ouverture (5) à travers laquelle la pointe d'outil (43) fait saillie est formée dans la portion de base (3), ledit canal (11) dans la fente dans la portion de base (3) se terminant au niveau de l'ouverture (5).

12. Outil chirurgical (1) selon la revendication 11, dans lequel la portion de base (3) comprend une section de nez verticale formée de manière intégrale (10) définissant l'extrémité de la poignée (6) dans laquelle l'ouverture (5) est formée.

13. Outil chirurgical (1) selon la revendication 12, dans lequel une extrémité de la portion de corps (4) comprend un retrait (16) pour recevoir la section de nez (10) de la portion de base (3).

14. Outil chirurgical (1) selon l'une quelconque des revendications précédentes, comprenant des moyens de coopération (15, 22) pour relier la portion de corps (4) à la portion de base (3).

15. Outil chirurgical (1) selon la revendication 14, dans lequel les moyens de coopération (15, 22) comprennent une paire de rails allongés (22) dépendant de la longueur de la portion de corps (15) pour l'engagement avec des retraits correspondants (15) dans la portion de base (3).

16. Outil chirurgical (1) selon l'une quelconque des revendications précédentes, dans lequel l'élément d'outil (7) est un composant métallique unitaire formé à partir d'une feuille plate par estampage, poinçonnage, découpe au laser, découpe à l'eau, érosion de fil métallique ou usinage.

17. Outil chirurgical (1) selon l'une quelconque des revendications précédentes, dans lequel la poignée (2) est pourvue de contours pour lui permettre d'être tenue à la manière d'un stylo entre le pouce et l'index et permettre un maniement léger précis.

18. Outil chirurgical (1) selon l'une quelconque des revendications précédentes, dans lequel la poignée (2) définit une cavité (54) en son sein et une section de paroi cassante (56) pour empêcher l'élément d'outil (7) de glisser dans la cavité (54).

19. Outil chirurgical (1) selon la revendication 18, dans lequel la paroi cassante (56) est configurée de sorte que lorsqu'une force est appliquée sur l'élément d'outil (7) dans une direction longitudinale vers la poignée (2), la paroi cassante (56) casse pour permettre à l'élément d'outil (7) de se retirer dans la cavité (54).

20. Outil chirurgical (1) selon la revendication 17, dans lequel la portion de base (3) ou la portion de corps (4) est colorée pour dénoter le type d'élément d'outil (7) contenu dans la poignée (2).

21. Outil chirurgical (1) selon l'une quelconque des revendications précédentes, dans lequel l'outil (1) peut être stérilisé pour être réutilisé.

22. Outil chirurgical (1) selon l'une quelconque des revendications précédentes, dans lequel la poignée moulée (2) est formée à partir d'un matériau plastique.
